**Europäisches Patentamt**

**European Patent Office**

**Office européen des brevets**

(19)

(11) Veröffentlichungsnummer : **0 139 278 B1**

(12) **EUROPÄISCHE PATENTSCHRIFT**

(45) Veröffentlichungstag der Patentschrift :
27.07.88

(51) Int. Cl.⁴ : **A 61·N 5/06**

(21) Anmeldenummer : **84112128.8**

(22) Anmeldetag : **10.10.84**

(54) **UV-Bestrahlungsgerät.**

(30) Priorität : **11.10.83 DE 3336939**

(43) Veröffentlichungstag der Anmeldung :
**02.05.85 Patentblatt 85/18**

(45) Bekanntmachung des Hinweises auf die Patenterteilung : **27.07.88 Patentblatt 88/30**

(84) Benannte Vertragsstaaten :
**AT BE CH FR GB IT LI NL SE**

(56) Entgegenhaltungen :
**DE-A- 2 315 721
DE-A- 2 547 949
DE-A- 3 035 624
GB-A- 1 468 556
US-A- 2 397 757**

(73) Patentinhaber : **Saalmann, Gerhard
Werrestrasse 94
D-4900 Herford (DE)**

(72) Erfinder : **Saalmann, Gerhard
Werrestrasse 94
D-4900 Herford (DE)**

(74) Vertreter : **Hoefer, Theodor, Dipl.-Ing.
Kreuzstrasse 32
D-4800 Bielefeld 1 (DE)**

**EP 0 139 278 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Bestrahlungsgerät mit einem mit Handgriff versehenen Gehäuse und einem darin angeordneten, von einem Reflektor umgebenen UV-Strahler (UV-Brenner) zur Erzeugung ultravioletter Strahlung für die Anwendung im medizinischen Bereich. Ein Gerät dieser Gattung ist aus US-A-2 397 757 bekannt. Es ist bekannt, daß an Schuppenflechte (Psoriasis) leidende Personen einen gewissen Grad von Besserung ihrer erkrankten Hautstellen erreichen, wenn sie sich der Sonne im Hochgebirge oder in bestimmten See-Bereichen aussetzen.

Es ist auch bekannt, daß eine hervorragende Besserung der an Psoriasis oder anderen Dermatosen erkrankten Hautstellen durch die Bestrahlung mit ultraviolettem Licht gewisser Wellenlänge erzielt werden kann. Die Behandlung der behaarten Körperstellen (mit längerem Haar), vornehmlich der behaarten Kopfhaut, mit ultravioletten Strahlen ist insofern schwierig, als die Haare die Strahlen nicht bis zu den Hautstellen hindurchlassen. Solche Hautstellen konnten also nur durch einzelnes Scheiteln des längeren Haares anschließende Bestrahlung durchgeführt werden, wobei die Strahlen möglichst senkrecht auf die Haut auftreffen sollen. Insbesondere bei langem Haar ist es schwierig und zeitaufwendig, die Kopfhaut zu bestrahlen. Hierfür wird im allgemeinen die Hilfe einer zweiten Person benötigt.

Der Erfindung liegt die Aufgabe zugrunde, ein handliches, kostengünstiges Bestrahlungsgerät in kompakter Bauweise zu schaffen, das es ermöglicht, behaarte Körperstellen (mit längeren Haaren) mit ultravioletter Strahlung zu behandeln, wobei die erkrankte Person die Behandlung in einfacher Weise selbst durchführen können soll.

Diese Aufgabe wird bei einem Bestrahlungsgerät der angegebenen Gattung erfindungsgemäß dadurch gelöst, daß eine kammähnliche Verzahnung frei vorstehend beidseitig der Stablampe an den freien, gegenüber der UV-Stablampe nach außen vorstehenden Kanten bzw. Schenkeln des Reflektors angeordnet ist, und parallel sich über die Länge der Stablampe erstreckt. Diese vorstehenden Verzahnungen (Zähne) zerteilen die parallelliegenden Haare und bilden damit freie Stellen auf der Kopfhaut, so daß die UV-Strahlen auf diese in vorteilhafter Weise auftreffen können. Die erkrankte Person kann das Bestrahlungsgerät selbst über die behaarten Körperflächen führen und durch den geringen Abstand von diesen Flächen die Isolationswirkung der Haare verringern.

Bevorzugt ist der Reflektor im Querschnitt U-förmig ausgebildet. Er ist an seinen freien Kanten bzw. U-Schenkeln auf der Länge der Stablampe mit einer kammähnlichen Verzahnung (einem Wellenschliff) versehen. Auf diese Weise wird beim Bestreichen der behaarten Haut mit dem Bestrahlungsgerät das Haar in parallele, ausreichend breite Furchen geteilt, so daß die Kopfhaut am Furchengrund jeweils eine intensivere Strahlung erhält.

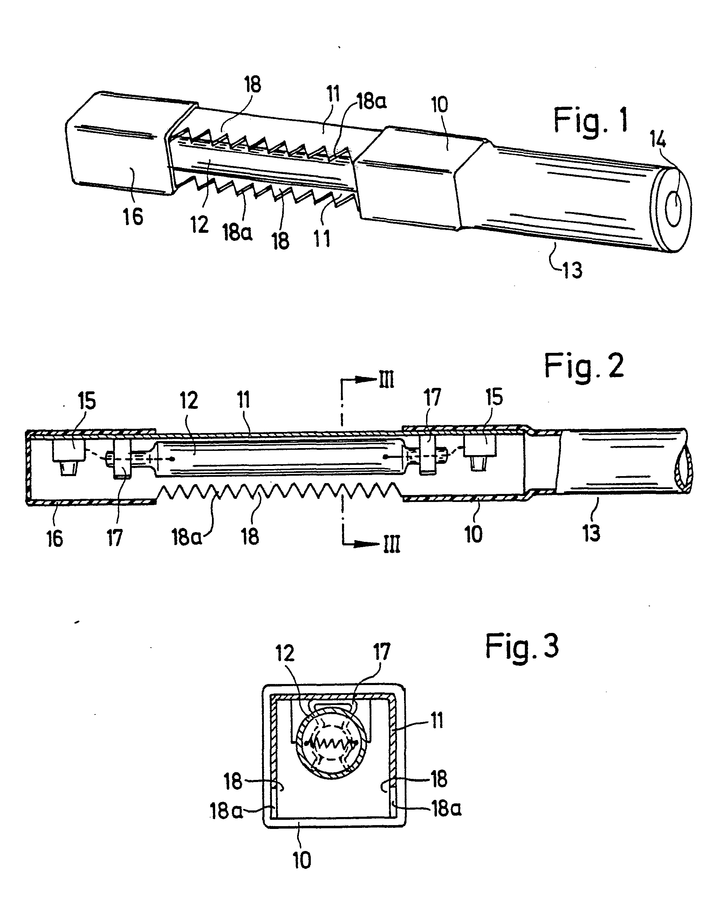

Es ist vorteilhaft, die einzelnen Zähne der Doppel-Verzahnung paarweise in Bewegungsrichtung des Bestrahlungsgerätes (in einer Ebene quer zu der Längsrichtung des Strahlers) im Abstand voneinander anzordnen (Fig. 1), so daß zwei Zähne hintereinander die parallelliegenden längeren (oder langen) Haare über den Abstand der Zähne voneinander zu längeren Furchen zerteilen.

Es ist aber in vorteilhafter Weise auch möglich, die in Bewegungsrichtung hintereinander vorstehenden Zähne (einer Doppelverzahnung Fig. 1) gegeneinander zu versetzen, so daß die Haare schlangenlinienförmig unter Bildung von Furchen bewegt werden.

Es ist weiterhin vorteilhaft, daß die Spitzen der Zähne etwa 15-30 mm gegenüber der Glashülle des UV-Brenners vorstehen, so daß demtentsprechend eine unmittelbare (nahe) Bestrahlung sich zeigt.

Ein Ausführungsbeispiel der Erfindung ist in der Zeichnung dargestellt und wird im folgenden näher beschrieben. Es zeigen :

Fig. 1 eine perspektivische Ansicht eines erfindungsgemäßen Handbestrahlungsgerätes ;

Fig. 2 einen Längsschnitt durch dasselbe Bestrahlungsgerät ;

Fig. 3 einen Querschnitt gemäß Linie III-III der Fig. 2 durch dasselbe Bestrahlungsgerät ;

Innerhalb eines Gehäuses 10 ist ein Reflektor 11 mit einem Ende befestigt, der eine Stablampe (Stabbrenner) 12 umgibt. Dieses Gehäuse 10 kann ein metallischer oder aus Kunststoff hergestellter Hohlkörper sein, der einen rechteckförmigen Querschnitt im Bereich des Reflektorendes aufweist. Der Reflektor 11, der die Form eines nach unten geöffneten U im Querschnitt aufweist, ist mit seinem Ende passend in das Gehäuse 10 eingesteckt und dort innerhalb des Gehäuses 10 befestigt. Der Reflektor 11 ist vorzugsweise ein spiegelndes Metallband mit hohem Reflektionsvermögen an der Oberfläche.

Das Gehäuse 10 ist mit einem Hangriff 13 einstückig ausgeführt, der sich an dem von dem Reflektor 11 abgewandten Ende des im Querschnitt rechteckigen, insbesondere quadratischen Teils des Gehäuses 10 anschließt. Der Handgriff 13 ist im Querschnitt kreisringförmig und weist an seiner von dem Reflektor 11 abgewandten Stirnseite eine Öffnung 14 auf, durch die ein Stromzuführungskabel in das Gehäuse 10 zu einer Anschlußklemme 15 für die Stablampe 12 hindurchgeführt ist.

An dem von dem Gehäuse 10 abgewandten Ende des Reflektors 11 ist auf diesen eine Schutzkappe 16 passend aufgesteckt, die im Querschnitt ebenso, wie das Gehäuse 10, rechteckförmig, insbesondere quadratisch, ausgebildet ist. Diese Schutzkappe 16 kann ebenso, wie das Gehäuse 10 mit dem Handgriff 13, ein Kunststoffteil oder ein Metallteil sein. Innerhalb der Schutzkappe 16 ist der Reflektor 11, ebenso, wie in dem Gehäuse 10, befestigt und für die Stablampe 12 ist auch in der Schutzkappe 16 eine Anschlußklemme 15 angebracht.

Die Stablampe 12 wird in dem Reflektor 11 von zwei Lampenfassungen 17 gehalten, die jeweils die Form eines Clips haben. An jedem Ende ist die Stablampe 12 in diesen Lampenfassungen 17 eingesteckt.

In den Ausführungsbeispielen liegt der Reflektor 11 zwischen dem Gehäuse 10 und der Schutzkappe 16 frei. Es ist aber auch eine Ausführung möglich, bei der der Reflektor 11 bis auf seine Austrittsöffnung vollständig von einem Gehäuse umgeben sein kann. Die nach unten weisenden freien Schenkel des U-förmigen Reflektors 11 sind mit einer kammartigen Verzahnung 18 versehen, so daß der Reflektor 11 wie ein Kamm wirkt, wenn er über die behaarte Kopfhaut mit längeren Haaren geführt wird.

Die Verzahnung 18 bildet dabei vorübergehend Furchen in dem Haar, so daß die aus der Stablampe austretenden UV-Strahlen am Grund dieser Furchen besonders gut in die erkrankte Kopfhaut eindringen können.

Das erfindungsgemäße Bestrahlungsgerät, dessen Stablampe 12 zur Erzeugung der ultravioletten Strahlen eine Hochdruck- oder Niederdruck-Quecksilber-Dampflampe sein kann, ermöglicht es, insbesondere durch die Anordnung des Handgriffs 13 und/oder die kurze Länge der vorstehenden Zähne 18a, Strahlungsenergie aus kürzestem Abstand auf die Haut aufzubringen, wobei eine besondere, die Behandlung durchführende Person nicht erforderlich ist. Die erkrankte Person kann mit einer Hand die Bestrahlung der Haut mit Hilfe des Bestrahlungsgerätes selbst durchführen.

Die Ausführung des Reflektors ist nicht auf eine im Querschnitt U-förmige Form beschränkt ; es können auch Reflektoren verwendet werden, die im Querschnitt beispielsweise halbkreisförmig oder in anderer Form gekrümmt ausgeführt sind.

**Patentansprüche**

1. Bestrahlungsgerät zur Erzeugung ultravioletter Strahlung zur Behandlung des behaarten Kopfes von Personen mit jeweils einer von einem Reflektor (11) umgebenden UV-Stablampe (12), die in einem mit einem Handgriff (13) versehenen Gehäuse mit einer seitlichen Strahlenaustrittsöffnung mit daran vorstehenden, kammähnlichen Zähnen (18) gehalten ist, dadurch gekennzeichnet, daß die kammähnliche Verzahnung (18) frei vorstehend beidseitig der Stablampe (12) an den freien, gegenüber der UV-Stablampe (12) nach außen vorstehenden Kanten bzw. Schenkeln des Reflektors (11) angeordnet ist, und sich parallel über die Länge der Stablampe (12) erstreckt.

2. Bestrahlungsgerät nach Anspruch 1, dadurch gekennzeichnet, daß die Zähne durch Wellenschliff gebildet sind.

3. Bestrahlungsgerät nach den Ansprüchen 1 oder 2, dadurch gekennzeichnet, daß der Reflektor (11) im Querschnitt U-förmig oder halbkreisförmig ausgebildet ist.

4. Bestrahlungsgerät nach den Ansprüchen 1 bis 3, dadurch gekennzeichnet, daß die Zähne der Verzahnung (18) jeweils paarweise in einer Ebene quer zu der Längsrichtung der UV-Stablampe (12) angeordnet sind.

5. Bestrahlungsgerät nach den Ansprüchen 1 bis 4, dadurch gekennzeichnet, daß der Reflektor (11) mit Ausnahme seiner zwischen den freien Kanten bzw. U-Schenkeln befindlichen Austrittsöffnung vollständig von dem Gehäuse umgeben und mit einem seiner über die Stablampe (12) hinausragenden Enden in dem Gehäuse (10) eingesteckt und dort befestigt ist, und daß der Reflektor (11) an seinem über die Stablampe (12) hinausragenden und von dem Gehäuse (10) abgewandten Ende mit einer dort befestigten Schutzkappe (16) abgedeckt ist.

6. Bestrahlungsgerät nach den Ansprüchen 1 bis 5, dadurch gekennzeichnet, daß das Gehäuse (10) mit dem in Bezug auf die Stablampe (12) dahinter angeordneten Handgriff (13) als einstückiger Hohlkörper ausgebildet ist, wobei an der freien Stirnseite des Handgriffs (13) eine Öffnung (14) für die Einleitung eines Stromanschlußkabels vorgesehen ist.

**Claims**

1. Irradiation device for producing ultraviolet radiation for the treatment of the hairy scalp of persons, having a UV irradiation lamp (12) surrounded by a reflector (11), which lamp is retained in a housing provided with a handle (13) and with a lateral outlet opening for the rays having projecting comb-like teeth (18), characterised in that the comb-like toothing (18) is arranged, projecting freely on both sides of the irradiation lamp (12), on the free edges or lateral sides of the reflector (11) projecting outwardly opposite the UV irradiation lamp (12) and extends parallel over the length of the irradiation lamp (12).

2. Irradiation device according to claim 1, characterised in that the teeth are formed as a wave-cut.

3. Irradiation device according to claims 1 or 2, characterised in that the reflector (11) is formed with a U-shaped or semicircular cross-section.

4. Irradiation device according to claims 1 to 3, characterised in that the teeth of the toothing (18) are arranged in pairs in a plane transverse to the longitudinal direction of the UV-irradiation lamp (12).

5. Irradiation device according to claims 1 to 4, characterised in that the reflector (11) with the exception of its outlet opening located between the free edges or U-sides is completely surrounded by the housing and with one of its ends projecting beyond the irradiation lamp 12 is inserted in the housing (10) and secured there, and in that the reflector (11) is covered on its end which projects beyond the irradiation lamp (12) and is distant from the housing (10) with a protective cap (16) secured there.

6. Irradiation device according to claims 1 to 5, characterised in that the housing (10) is formed as a unitary hollow body with the handle (13) arranged at the rear relative to the irradiation lamp (12), wherein on the free front side of the handle (13) an aperture (14) is provided for the introduction of a current connection cable.

**Revendications**

1. Appareil de radiothérapie en vue d'émettre des rayons ultraviolets pour le traitement de personnes à tête chevelue, cet appareil comportant chaque fois une lampe torche à ultraviolet (12) entourée d'un réflecteur (11) et maintenue dans un logement pourvu d'une poignée (13) et comportant une ouverture latérale pour la sortie des rayons et sur lequel des dents (18) ressortent en saillie sous forme d'un peigne, caractérisé en ce que les dents sous forme de peigne (18) sont disposées librement en saillie de part et d'autre de la lampe torche (12) sur les bords ou les ailes libres du réflecteur (11) ressortant en saillie vers l'extérieur vis-à-vis de la lampe torche à ultraviolet (12), tout en s'étendant parallèlement sur la longueur de cette lampe torche (12).

2. Appareil de radiothérapie selon la revendication 1, caractérisé en ce que les dents sont formées par meulage ondulé.

3. Appareil de radiothérapie selon la revendication 1 ou 2, caractérisé en ce que le réflecteur (11) est réalisé avec une section transversale en U ou en demi-cercle.

4. Appareil de radiothérapie selon les revendications 1 à 3, caractérisé en ce que les dents du système (18) sont chaque fois disposées par paires dans un plan transversal au sens longitudinal de la lampe torche à ultraviolet (12).

5. Appareil de radiothérapie selon les revendications 1 à 4, caractérisé en ce que, à l'exception de son ouverture de sortie situé entre les ailes de l'U ou les bords libres, le réflecteur (11) est entouré complètement par le logement (10) dans lequel il est enfiché et fixé par une de ses extrémités ressortant au-delà de la lampe torche (12) et en ce que le réflecteur (11) est recouvert, à son extrémité ressortant au-delà de la lampe torche (12) et éloignée du logement (10) par un chapeau protecteur (16) fixé à cette extrémité.

6. Appareil de radiothérapie selon les revendications 1 à 5, caractérisé en ce que, avec la poignée (13) disposée derrière la lampe torche (12), le logement (10) est réalisé sous forme d'un corps creux d'une seule pièce, une ouverture (14) pour l'introduction d'un câble de raccordement électrique étant prévue sur la face frontale libre de la poignée (13).

Fig. 1

Fig. 2

Fig. 3